# EUROPEAN PATENT SPECIFICATION

(11) **EP 2 872 332 B1**
(45) Date of publication and mention of the grant of the patent: **08.08.2018**
(21) Application number: 13732502.3
(22) Date of filing: 28.06.2013
(51) Int. Cl.: B32B 37/10, B29C 65/08, A61F 13/49, D04H 1/559

(54) **SANDWICH STRUCTURES WITH FREE FLOWING MATERIALS**
SANDWICHSTRUKTUREN MIT FLIESSFÄHIGER MATERIALIEN
STRUCTURES SANDWICH AVEC PRODUITS À ÉCOULEMENT LIBRE

(30) Priority: 29.06.2012 GB 201211577; 19.09.2012 GB 201216670
(43) Date of publication of application: 20.05.2015
(73) Proprietor: Concepts for Success - C4S e.K., 53881 Euskirchen (DE)
(72) Inventor: CHRISTOPH, Schmitz, 53881 Euskirchen (DE)
(74) Representative: Plischke, Manfred
(86) International application number: PCT/EP2013/063597
(87) International publication number: WO 2014/001488

(56) References cited:
- WO-A1-2011/080859
- WO-A1-2012/042055
- US-A1- 2002 016 122

## Description

### Field of the invention

The present invention is directed towards free flowing materials sandwiched between web materials as well as to the method and an apparatus for the continuous making of sandwiches structures. At least some of the web materials are bonded to each other at bonding points, which define bonding regions which delimit the accumulation regions in which the free flowing material is positioned.

### Background

Positioning free flowing material between other layered materials is a well known technology with very broad application areas.

A first application area relates to the adsorption of certain compounds of odours or other components of a liquid or gaseous fluid from this fluid by sandwiching a particulate adsorbent material.

According to JP2006263490A a filter medium used in air cleaner is obtained by carrying out lamination integration of cover material containing flame retardant on surface(s) of deodorizing particle layer consisting of deodorizing particles and connecting resin.

A cloth for ceilings or walls is described in JP2001303460A, comprising a non-woven fabric sheet containing charcoal, obtained by mutually adhering non-woven fabrics and carbon particles using. The use of adsorbent laminates for protective clothing is described e.g. in JP2002201566A and DE102004044347A1. DE10037862A1 (Carl Freudenberg) describes the making of a particle-filled textile laminate, for use e.g. in protective clothing and feminine hygiene products, which comprises central textile layer enclosing particles and two outer layers whose fibres are bound to it at points along their length.

The preparation of oxygen absorbing pads for keeping the quality of food is described in JP2002002602A.

Industrial use of adsorbing components of fluids is known from JP2004330631A, relating to an adsorption sheet for deodorizing liquid, having an adsorption particle layer consisting of adsorption particles, arranged between fibre layers.

Similarly, sandwiched particles may release certain compounds, such as described in US4952400, relating to a particle and fragrance capsule sampler comprising thin dry layer of powder on substrate.

Other applications relate to allowing substances contained in a sandwich to be released there from, such as described in WO2002102592A1 showing a laminate for being formed into e.g. self-standing bag or pouch or geotextile substitute, having predetermined wavelength value of waved flute configuration of minifluted ply. Also CN102152524A discloses a non-woven suture material filled with water hyacinth particles between non-woven materials. Similarly, in WO2001083669A1 (P&G) a pouched composition is used as cleaning composition such as laundry or dish washing detergent, comprising particulate components each of which forms fixed region in compartment.

Particulate applications use sandwich structures wherein the sandwiched material performs a physical action, such as shown in JP9029880A relating to a noise-absorbing laminate comprises composite solid covered with layer of felt. The making of shock and moisture absorbing pads is described in US6591580B1.

Significant effort has been put against creating sandwich structures adapted to allow liquid absorption such as in US5135787, wherein an iced food shipping container with aqueous liquid absorbing pad is described, including super absorbing polyester particles distributed in polymer carded web contained between hydrophilic fabric outer layers, or in WO1993020950A1 relating to an absorbent structure for absorbing fluids in e.g. food trays comprising super absorbent particulate carboxymethyl cellulose powder sandwiched between and adhesively secured to fluid permeable sheets in roll form.

Other applications in the area of handling of liquids aim at using sandwich structures as a barrier, see e.g. US5091234A for lining tanks, ponds or buildings such as subterranean walls by providing a bentonite layer resulting in swelling of the particles to provide a leakproofing action upon wetting through pervious sheets. In WO1991007319A1 an apparatus for sealing plastic sachets with particulate matter is described, wherein a metering roll places particulate matter between two webs and a heated roll seals the sachets.

A specific area of liquid handling relates to disposable absorbent articles. In partially related patent publications WO1995011651A1, WO1995011652A1, WO1995011653A1 and WO1995011654A1 (K-C; Tanzer et al.) absorbent articles with pockets for superabsorbent particles are described. US5156902A (K-C) describes intermittent distribution of particles in nonwoven webs to form zones of varying properties along the length of the web. US20050101217A1 (Paragon et. al; Deler et al.) shows dry forming of absorbent core composite of particulate matter deposited onto fibrous material, whereby both are encased between sheets by using a vacuum chamber,

Another series of partially related patent applications is EP1621165, EP1621166, and EP1621167 (P&G; Blessing et al.) describing among others the making of patterned sandwich structures of particular material, such as superabsorbent, between a carrier and a cover web by forming pockets into which particulate material is deposited. The carrier and cover webs are bonded in essentially particle free bonding regions.

Further methods for manufacturing absorbent composites for use in absorbent structure of absorbent article as described in WO2012052173A1 (Vynka Bvba; van Ingelgem) and WO2012048878A1 (ROMANOVA BVBA STARTER; VAN DE MAELE) showing the deposition of particulate material on carrier layer to create printing pattern of particulate material.

In particular when sandwiching swelling materials such as superabsorbent materials, it is desired that the sandwich structure does not impede the swelling. In WO1996016625A1 (P&G; Dragoo et al.) a process is described for manufacturing stretchable absorbent article using stream(s) of fibrous melt-blown elastomeric adhesive to entrap absorbent particulate material. WO2002053363A2 (K-C; Fish et al.) discloses a flexible laminate structure such as for disposable diaper, which has unfused portions defining pockets containing discrete regions of functional material. In WO2011/141009 laminate of superabsorbent material and webs is described which is elasticized by the use of elastic threads.

In the above applications, the bonding of the sandwich structures is often described as preferably being executed by adhesive bonding. For certain applications, adhesive free bonding has been mentioned, such as in WO'173 and WO'878, without specifically describing how ultrasonic welding can be performed. WO2008/065365 describes an ultrasonic sealing unit with an anvil roll comprising a raised sealing surface on its surface that can extend in a helical shape on this surface for interacting with the sonotrode. The axis of the helix is essentially parallel to the axis of the anvil roll. WO2012/042055 (C4S, Schmitz) describes a particularly effective approach with regard to ultrasonic bonding by employing a flexible anvil, which also allows for creating particular bonding patterns. The flexible anvil may preferably be executed as a helical anvil, such as a helical spring, mounted on the surface of a turret.

A further sandwich structure is known from patent publication US 2002/0016122 A1, comprising a carrier web and a cover web with a displaceable elastic layer positioned between them. A free flowing material in particulate form is not disclosed. However, there is still a need for improved sandwich structures with particular bonding requirements and for providing a flexible manufacturing method and a corresponding apparatus for the making of sandwich structures, in particular relating to particular patterns of the bonding regions as well as of the accumulation regions of the free flowing material.

### Summary

The present invention is directed to a sandwich structure with free flowing material between webs in accordance with the appended claims.

### Brief description of the Figures

Fig. 1A to E and Fig. 2A to D depict schematically bonding points and their relative positioning.
Fig. 3A to C show schematically an apparatus for the bonding with a helical element.
Fig. 4A and B show schematically a suitable arrangement of helical elements.
Fig. 5A and B depict schematically arrangements of helical elements and regions for depositing accumulations of free flowing material.
Fig. 6A to E show schematically further arrangements of helical elements and details for exemplary executions.
Fig. 7A to E show schematically an execution for rotatably mounted helical elements and the resulting effect.

Same numerals in various figures depict same, corresponding or equivalent elements.

### Detailed description

The present invention is directed towards free flowing materials sandwiched between web materials as well as to the method and an apparatus for the continuous making of sandwiches structures. The web materials are bonded to each other at bonding points, which define bonding regions which delimit the accumulation regions in which the free flowing material is positioned.

The selection of free flowing materials useful in the present invention is not particularly limited.

In a first execution, these can be fluids, including liquids over a wide range of viscosities up to being pasty or semi-solid, gels, dispersions, etc.. As a skilled person will readily realize, certain fluids put certain restrictions to the selection of web materials, such that for this execution films or fluid impermeable non-wovens should be selected.

In another execution, the free flowing materials can be bulk particulates, such as granules, powders and the like. Here, too, the web properties may need to be adapted, for example if powders are to be sandwiched, the pore size of non-woven materials may need special selection.

The term "regions" refers to a geometric x-y-dimensionally extending area delimited by a circumscribing line, which may be a continuous or solid line or which may be a noncontinuous line, such as a dashed or dash-dotted line. The circumscribing line may also result from two or more intersecting straight or curvilinear lines. Such a line may be physically present and/or be visible, or may be a thought line, such as when the region consists of two or more sub-regions, which can be circumscribed by an envelope line. An envelope line is the line around a set of sub-regions with the shortest line length. Regions or lines may also be essentially endless, such as is the case with closed line on the surface of a cylinder.

It should be noted, that - unless expressly stated - within the present context the Cartesian system is also applied to surfaces of e.g. cylinders, which strictly speaking have a curvature. However, when the diameter of the cylinder is sufficiently large compared to the other geometries on the surface, e.g. an indentation towards the centre of the cylinder, it is considered a "z-directional" indentation, i.e. neglecting the curvature of the cylinder surface. Thus, a region may exhibit a z-directional extension, such as when an x-y-directionally extending web is deflected z-directionally. Accordingly, the x-y-surface extends over the surface of such a cylinder.

Within the present context, three regions are to be distinguished:
"Accumulation regions" are regions where free flowing material is accumulated;
"Bonding regions" are regions in which the webs are bonded together, as will be discussed in more detail below. The bonding regions comprise consolidation points or regions, where the web materials are melt-fusion bonded. Thus, the circumscribing line of a bonding region will circumscribe at least two consolidation points and unbonded regions there between.
"Empty regions" are regions where essentially no free flowing material is present, but which do not belong to a bonding region;

The sandwich structure according to the present invention is made of a plurality of accumulation regions, also referred to as "pockets", in which the free flowing material is positioned.

The term "accumulation of free flowing material" describes that a minimum amount of free flowing material is present and this amount is discernible in this region as compared to a bonding region or an empty region. For example in case of the free flowing material being in particulate form, "accumulation" means that more particles per unit area are present in the filled region as compared to the other regions.

When the amount of free flowing material is expressed as "basis weight", determined by the weight of the material per unit area and expressed in "gram / m²", a sandwich structure has an overall average basis weight of the free flowing material, which is lower than the local basis weight in an accumulation region, but higher than in a bonding region or an empty region. However, even within an accumulation region the basis weight does not need to be constant over the full region, but there can be variations within the region, as can be determined by conventional methods such as weighing sectional regions, or x-raying, or under certain circumstances optically. If necessary, the basis weight distribution may be approximated by a smoothed curve when following a cross-sectional view through this heap. An accumulation region may have various forms and shapes, such as oval or elliptic or may be irregularly shaped. The dimension of an accumulation region depends on the intended use, and will often be more than 1 mm, more than 2 mm but often less than 50 mm, or less than 25 mm along any direction. The distance between two neighbouring accumulation regions in a pattern may often be more than 1 mm, or more than 3 mm, but often less than 30 mm, less than 15 mm, and even less than 10 mm. If the separation between two neighbouring accumulation zones is accomplished by a separation line which is in the form of a sequence of bonding points or regions, there may actually be zones between two neighbouring bonding points like channels between two neighbouring accumulation zones. Whilst it is not preferred, SAP material may also be positioned in such channels.

Generally, the selection of web materials will primarily depend on the in-use requirements and can be done over a wide range of materials.

The term "web material" refers to a material, which is essentially endless in one direction, i.e. the longitudinal extension, or the length, or the x-direction in Cartesian coordinates relative to the web material. Included in this term is an essentially unlimited sequence of pieces cut or otherwise separated from an essentially endless material. Typically, the web materials will have a thickness dimension (i.e. the z-direction) which is significantly smaller than the longitudinal extension (i.e. in x-direction). Typically, the width of web materials (the y-direction) will be significantly larger than the thickness, but less than the length. Often, though not necessarily, the thickness and the width of such materials is essentially constant along the length of the web. Without intending any limitation, such a web material may be a film material, a woven material, a nonwoven material, web material comprising cellulosic material such as a tissue or any combination thereof. Typically, though not necessarily, web materials are supplied in roll form, or on spools, or in a folded state in boxes. The individual deliveries may then be spliced together to form the essentially endless structure. A web material may be composed of several web materials, such as multilayer non-woven, coated tissues, non-woven / film laminates. In case of a combination of several web materials, these may have co-terminating dimensions, or different widths (i.e. one or more of these webs extend laterally outwardly of others or CD positioning (i.e. one of the webs is positioned cross-directionally offset of another one). A web may also comprise another web material only in machine-directionally offset regions, such as when a series of patches of a second web is combined with the primary, continuous web. The combination of the web materials to form a carrier or cover material may comprise the pre-bonding of the individual web forming the combined web. Alternatively, they may be merely brought in the appropriate positioning and may be combined by the bonding of cover and carrier material. Web materials may comprise other materials, such as added binding material, particles, hydrophilizing agents and the like.

Apart from further satisfying general requirements for good processing, such as exhibiting sufficient strength, retention of the free flowing material, avoiding dustiness, etc., the web materials should allow bonding of and to each other.

In a first execution, the webs can be bonded to each other by means of an adhesive or glue. To this end, either the materials and/or the glue should be formulated so as to provide appropriate surface properties to allow a good bond.

In another, not necessarily exclusive execution, the bonding can be in the form of melt fusion bonding. Thus, in a first variant of this execution, both webs may comprise or essentially consist of thermoplastic material or melt fusionable material with melt temperature ranges approximately in the same range. Each of the webs may additionally comprise higher melting range materials, or even materials not exhibiting thermoplastic properties. However in these cases should be overall sufficient meltable material available so as to perform the melt fusioning, such as when non-thermoplastic material has sufficient interstices or voids into which the molten compound(s) of the other may penetrate into to accomplish the bonding.

In another variant of this execution, a first of the webs may not have melt fusionable compounds, if the other web compensates such as by having sufficient basis weight to produce a bonding region, such as when material of the other web can penetrate into the first one. Thus, non plastic compounds like cellulose or starch based materials combined with heat bondable compounds or components may be used. Thus a particular execution may comprise a cellulose based material. Such a material may be positioned between two webs comprising thermofusible material or in a layered configuration on the outside of the sandwich forming webs, or such a material may form one of the sandwich forming webs, if the other web contributes sufficient thermofusible material. It should be noted, In order to allow formation of "pockets" in which the free-flowing material is deposited and accumulated, the carrier web exhibits preferably a good pliability, such as by exhibiting a Circular Bend Flexibility of less than 50 N, more preferably less than about 20 N, even more preferably less than 5 N, and even flexibility values of less than 1 N may be suitable for certain applications, when tested according to ASTM D4032-94 (Standard Test Method for Stiffness of Fabric by the Circular Bend Procedure) with a 100 N load cell and a 100 mm/ min crosshead motion, e.g. when using a MTS Q-Tester with Testworks 4 software, as available from MTS System Corp., Minnesota, USA.

For certain executions, it is preferable that the carrier web exhibits an extensibility, as can be determined by assessing the elongation at break by well known methods, in its CD or MD direction of at least 5%, preferably more than 10%, and even more preferably more than 100%, and preferably less than about 200%.

For particular executions the carrier web is preferably air-permeable. In case of the free flowing materials being in a fluid state during manufacturing and/or storage or transportation, the webs are preferably essentially impermeable to the fluid. "Essentially impermeable" refers to one property or a set of properties which prevent unintended penetration of the free flowing material to the outside, such as may be achieved by using fluid impermeable films, or hydrophobized, small pore size webs in case of aqueous liquids, etc..

In a preferred execution, the carrier web is a nonwoven material, such as a carded, wet- or airlaid web, or a melts-spun web, which may be a single layer or which may comprise several layers of melt-spun, spunbonded, and / or melt-blown webs, and may be denoted as "SMS", "SMMS", SSMMS, ... webs, wherein "S" stands for spunbonding and "M" for melt-blowing.

Optionally, though often not preferred for processing or for economical reasons, at least one of the surfaces of one or both of the webs may comprise a surface layer which can function like an adhesive or compatibilizer.

Within the present context, the term "bonding" refers to connecting webs essentially permanently, such that typically at least one of the webs will by irreversibly damaged if the bonding shall be opened. A first execution may employ suitable adhesives, such as hot-melt adhesives. A further execution is thermal consolidation by melt fusion or at least thermoplastic deformation of at least a portion of one or more web(s).

This bonding is performed in bonding points, which denotes either a region of adhesive connecting the two webs or a region of thermoplastic deformation in one or both webs simultaneously. Both approaches are generally known in the art, the latter one for example for consolidating nonwoven webs by a calender roll or for connecting webs by pressure or ultrasonic bonding.

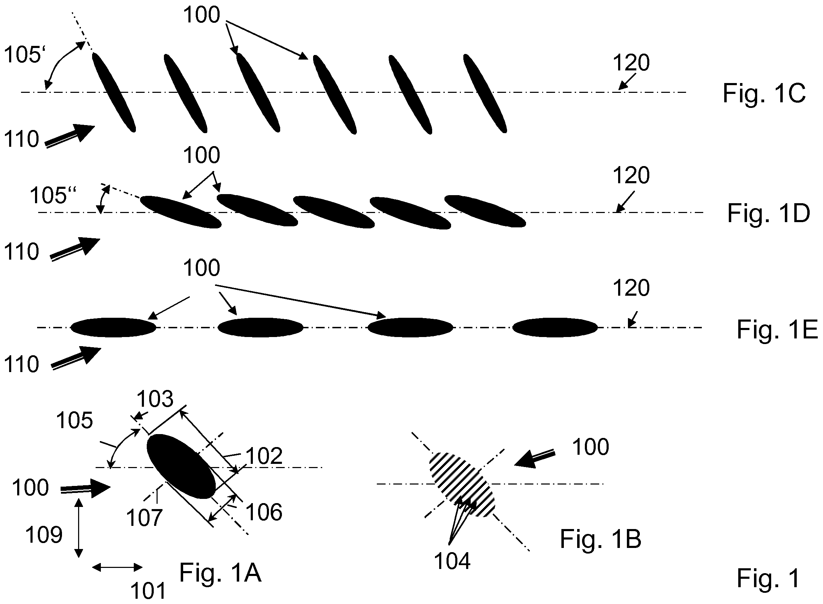

Referring to Fig 1A a bonding or bond point exhibits a bonding point centre, defined as the geometric balance point of the bonding point. Further, each bonding point 100 exhibits a length axis 103, which is the line connecting the most further apart points through the centre bonding point centre, and a short axis 107 which can also be described as the shortest line running through the bonding point centre and connecting two opposite points of the perimeter of the bonding point. Typically, though not necessarily, the long and the short axes cross at a right angle. Along these axes a bonding point exhibits a length 102 and a width 106 extension, whilst the z-direction corresponds to the thickness direction of the web. The relation of the length to the width of the bonding point is described by the aspect ratio. A bonding point further exhibits an orientation, defined as the smallest angle 105 of the long axis to the length direction 101 of the web, which is typically aligned with the machine direction.

As can be seen in Fig. 1B, and described in more details in the above mentioned WO'055 publication, to which express reference is made, bonding points forming the bonding pattern may be formed of bonding point sub-structures, such as when a generally elliptical bonding point is made of parallel bonding stripes 104, each of which end at the enveloping line, here shown as an ellipsis.

A particular execution of a bonding point is a bonding line exhibiting a certain, but not necessarily constant, bonding line width, and which may be straight or curvilinear. A bonding line may have a limited bonding line length, or may be essentially endless, i.e. being limited by the dimensions of the structure. A bonding line may consist of several bonding line elements, lined up along the general direction of the bonding pattern line. Often the web comprises a plurality of individual bonding points forming a patterned bonding region. Fig 1C shows a patterned bonding region 110 in the form of a series of bonding points 100. The connecting line of the centre points of respectively adjacent bonding indentations form a bonding pattern line 120. The bonding points may be at various angles to the pattern line, as indicated in Fig. 1D, and may actually be aligned therewith (Fig. IE).

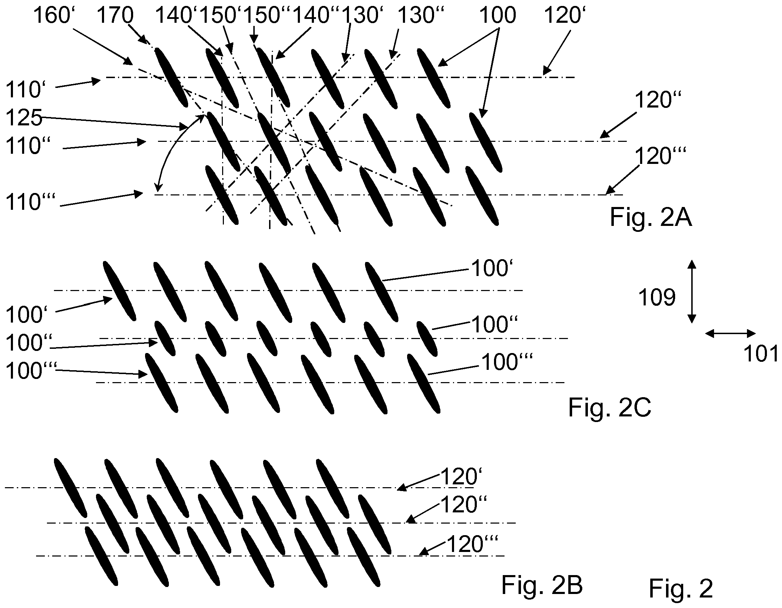

Fig. 2A shows for explanatory purposes three of the bonding point series as in Fig. 1C in a parallel and equidistant adjacent position, i.e. all bonding points are of equal size and angle. As can be seen, such a pattern now defines several sets of pattern lines at several angles to a reference line of the web. This is indicated by the lines 120', 120", 120'" corresponding to the line in Fig. 1C, and by line sets 130', 130" , ..., 140', 140", ..., 150', 150", ..., 160', 160", 170, .... As shown in Fig. 2A a pattern angle 125 can be defined as the smallest angle between line 120 and the one of the sets 130, 140, ... which is determined by connecting the centre point of a bonding point with the centre point of the closest neighbouring bonding point, in Fig. 2A line 170.

If several pattern lines are parallel and straight lines, a "column" of bonds on a nonwoven web can be defined as a group of nearest neighbouring bonds of like shape and rotational orientation that are arranged along the line that exhibits the smallest angle with a reference line, here shown as in the length direction of the web, typically aligned with the machine direction. Similarly, a "row" of bonds on a nonwoven web is a group of bonds of like shape and rotational orientation that are arranged along a line that exhibits the smallest angle to the width direction of the web typically aligned with the cross machine direction.

As indicated in Fig. 2B the distance between two adjacent pattern lines 120', 120", 120'" may be such that an intermeshing or interpenetrating pattern may be formed. In a further execution (see Fig. 2C), there may be two or more types of bonding points, e.g. 100' and 100", each forming e.g. a first and a second bonding sub-pattern, which may interpenetrate or intermesh each other, jointly forming the composite bonding pattern.

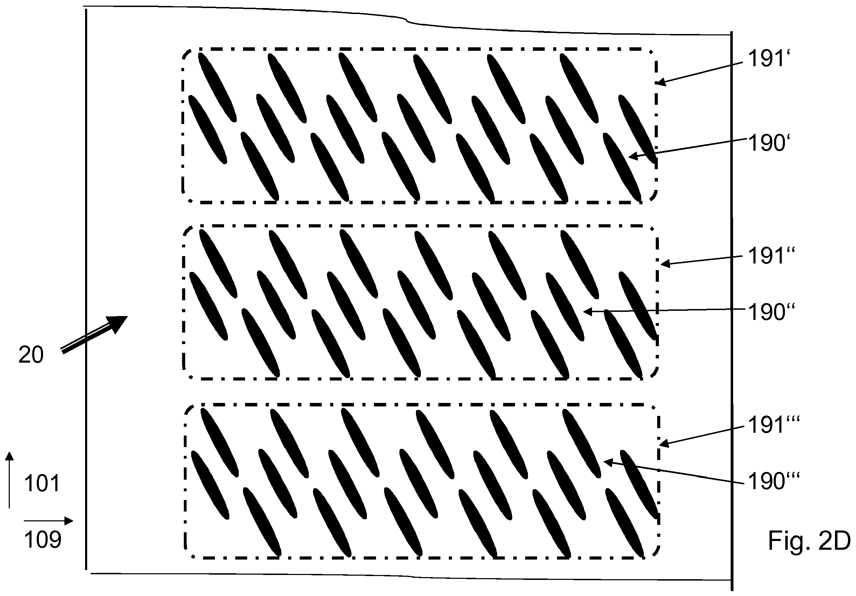

In yet a further execution (see Fig. 2D), a plurality of bonding points (of the same or of different type) may form a macro-pattern 190', 190", 190'", which is repeated in MD- or CD- direction of the web 20. A typical application for such a macro-pattern is a repeating series of absorbent articles, each comprising a (single or composite) bonding point pattern.

The present invention allows to create very specific bonding patterns by modifying one or more of the following parameters of the bonding pattern
- size and shape of bonding points, i.e. length, width and aspect ratio;
- length / width distance, which may be uniform or not;
- angle of pattern lines;
- angle of bond indentation;
- size and aspect ratio of bonding indentation.

Preferably, the bonding points exhibit an aspect ratio of their long axis to their short axis of at least 1.05 to 1, preferably of 1.1 to 1, and even up to 200: 1. In a preferred embodiment, the long axis of the bonding points is positioned such that its smallest angle to the pattern line is an angle of less than 90°. In case of a curvilinear pattern line, the angle is determined versus a tangent of the pattern line in the bonding point centre.

The distance of the bonding points along the pattern line may be less than about half a millimetre or more than 50 mm, depending on the desired degree of barrier function of the bonding pattern and/or the immobilization function with regard to the free flowing material.

Within the present invention, the bonding points may be formed by conventional means, such as adhesive application or melt fusion bonding.

A particular execution is achieved by heat- or melt-fusing the webs together, as well known as such in the art. To this end, the webs are guided through a gap formed by a pair of anvils whilst or after the temperature of at least one, often of both of the webs is or has been raised by an energy source to the softening, often the melt temperature of at least one of the compounds of one of the webs. Upon application of at least some pressure the softened or molten compounds form the bonding points.

Energy sources may be alone or in combination:
- pre-heated web material;
- the pressurizing as such by a pair of rolls acting under high pressure ;
- heated (often patterned) bonding roll(s);
- non-contact or indirect heating, such as by hot-air blowing or radiation heating;
- a preferred execution is sonic, more preferably ultrasonic energy emitted by a "sonotrode", optionally a rotating sonotrode, such as described in US20050034820 (Herrmann US, Schneider)

Typically, the pair of anvils is made of the energy source and a counter acting anvil, though the distinction between "energy source" and "anvil" may in some instances be blurred, such as when two non-heated anvils are forming the gap and the energy has already been transferred to the web(s) or is induced by pressing the anvils together. Similarly, the term "counteracting" should not be seen limiting, but rather also to encompass the situation of the energy source being the first anvil and essentially stationary and the second "counteracting" anvil being moveable.

Within the present invention and as will be discussed in more detail, the "second" one of the anvils is the first web support system and this is considered as the "counteracting" anvil. Typically, the opposed "first" anvil is the energy source, whilst at least some of the energy may also come through the first web support system, or from another energy source.

In the case of using adhesive bonding, the term "anvil" is to be understood as a support for establishing good contact between the webs.

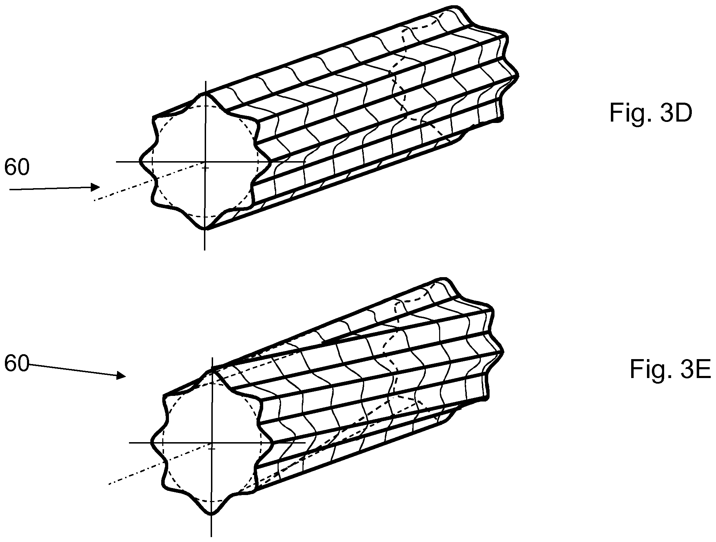

The surface of the first anvil, which is oriented towards the second anvil, may have various shapes adapted to the desired interaction with the second anvil and the web between the anvils to create a desired predetermined bonding pattern. Thus, the first anvil may have a flat surface, such when considering a conventional sonotrode, or it may have a cylindrical surface, such as when considering a well known rotating sonotrode. The surface may also be concavely shaped, e.g. when the anvil surface follows a circumscribing circle and it is desired that the contact region is enlarged or the contact period prolonged. If particular bonding patterns are desired, the surface may also exhibit particular shapes, having a chevron like shape or a wavy surface, as indicated in Fig. 3D and 3E.

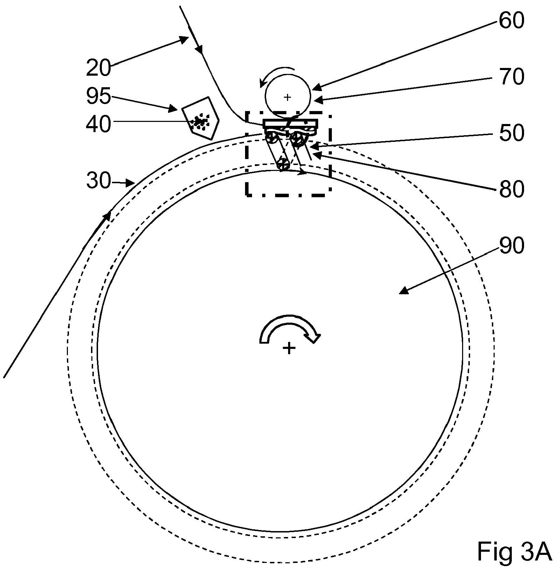

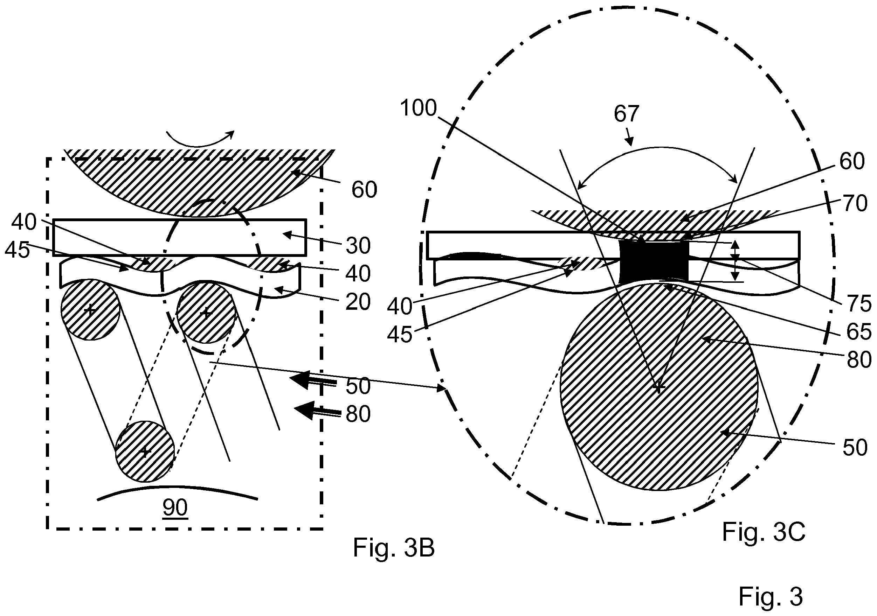

Considering the schematic presentation of an apparatus in Fig. 3 for non-limiting explanatory purposes, a carrier web 20 and a cover web 30 are shown with some free flowing material 40 there between as provided by a supply means 95. Also shown is an energy source 60, such as a rotating sonotrode, functioning a as first anvil 70 and a second anvil 80, here in the form of helically wound wire 50 on a rotating support means (90), the two anvils 70 and 80 forming the gap 75, in which the two webs are melt fusion bonded forming a bonding point 100.

Similarly, the bonding may be achieved by adhesive bonding. To this end, adhesive may be applied to either the carrier or cover web such as by well known so called "kiss rolls" or spray application, optionally supported by directed air flow and/ or vacuum. The bonding points will then be formed by a compression of the first and second anvils together.

Generally, the surfaces of the anvils which compress the webs correspond to the bonding points of the web(s), whereby the bonding points will not necessarily exactly mirror the shape of the surfaces of the anvils

Within the context of the present invention the contact surfaces are portions of structures which exhibit a rounded profile in the z-x- and/or z-y-plane or any other plane perpendicular to the x-y-plane. When such a structure would be contacted by an essentially non-extensible web (such as a sheet of metal), the contact surface would be a point corresponding to the tangent point. When contacting the surface with an extensible web - such as a typical film or non-woven material made from a thermoplastic material such as polyethylene or polypropylene - and applying small z-directional forces such as a result from guiding the web over a turret, or by applying a z-directional deformation force, such as a vacuum, the web will deform over the first web support system and cover a larger area. In Fig. 3B, this is indicated by the angle 67. If, for example, at otherwise same conditions, the z-directional deformation of the web will be increased (i.e. the pocket "deepened"), also the contact area 65 between the web and the anvil will be enlarged and the angle 67 will open.

The perimeter and size of the bonding point in the web will approximately correspond to the projection of the bonding surface of the first web support means into the plane of the web. Thus, when looking in a top view at a first web support means (and neglecting certain curvature, e.g. of the support 90 of the sandwich forming element, as has been done in Fig. 1, 2, and 3), a contact surface pattern corresponds to a bonding pattern in the web, as discussed in the above. Similarly, in analogy to Fig. 2C, several contact surface pattern lines can be identified.

It should be noted that the match of the contact surfaces and of the bonding point shape depends strictly speaking at least on the following aspects:
1) the shaping of the first web support system,
2) the properties of the webs (thickness, and to a certain extent extensibility);
3) the shaping of first anvil, e.g. the sonotrode;
4) the process conditions, such as vacuum or air flow, web tensions, etc.

Considering for explanatory purposes the first of these aspects only, namely the shape of the first web support means according to the present invention, this shape forms a pattern of distinct bonding points along a pattern line, which may be a straight line (such as in a row or column), but which may also be curvilinear.

In preferred executions the first web support system comprises several first web support members, each providing a particular bonding pattern and a bonding pattern line. If several bond patterns lines are positioned parallel but offset to each other, they may form a pattern which looks like the pattern in Fig. 2. As will be described herein below, several bond pattern lines may also cross each other or form a macro bond line, such as when a pattern line is interrupted (e.g. when intersecting another pattern line) and continued thereafter.

Such a bonding pattern can readily be achieved by employing bonding means as described in more detail in the above referenced WO'055-publication, to which express reference is made.

In a particularly preferred embodiment the first web support comprises flexible, preferably helical elements.

A helix is a three-dimensional curve that turns around an axis at a constant or continuously varying distance while moving parallel to the axis., such as well known coil spring, i.e. a spring which can be made by winding a wire around a cylinder. Generally, such helical springs can be made and used as compression springs which are designed to become shorter when compressed along their length direction. Their turns (loops) are not touching in the unloaded position. Tension or extension springs are designed to become longer under a pull force along their length direction. Adjacent wire turns (loops) may touch each other in the unloaded position. Such a helically wound wire is defined by the inner and outer diameter of the helix (or coil), the form of the wire, the diameter of the wire (particularly when the wire has a circular cross section), the pitch length (i.e. the distance of the centre points of adjacent wire turns), the canting angle (i.e. how much the connecting line of two adjacently opposed wire cross-section centre points is inclined versus the axis), and the hardness, stiffness and composition of the wire material, particularly at its outer surface. Typically, such helical elements show a circular cross section, but non circular cross sections such as e.g. elliptical ones may be desirable for particular applications. This also applies to the wire forming the helix, which may have a circular, a elliptical, or segmented cross-section, i.e. a circular or elliptical cross-section of which one or more segments are removed - either at the top surface (i.e. oriented towards the web(s)), and/or at one or both sides (i.e. oriented towards neighbouring wires). Thus a helical element useful in the present invention exhibits an axis that is essentially parallel to the surface of a turret, such as when a helix is wound around the surface of the turret.

Considering such a primary or first order structure of a straight helical anvil in Cartesian coordinates, the length or x-axis corresponds to the helix axis, whilst the y-direction is considered as width direction and the z-direction of thickness or height. In some embodiments, neighbouring wires forming the helix may displaceably contact each other. Preferably the wire has a rounded wire cross-section, more preferably elliptical, most a preferably circular one. Alternatively, a part of the surface may be flattened, such as by being filed off, or the wire may have an oval or half-oval cross-section. Typically, the wire is solid, but provided the required mechanical properties are met, it can also be executed as a hollow tubular wire, optionally further comprising a core material. A wire may actually also be formed in the form of a helix (i.e. a "zero order helix"), optionally being wound around a flexible core, such as a hexagonal core. Any of the helical structures may be right- or left-handed.

Optionally the flexible elongated member can be made from a primary helical structure formed by a secondary helical structure, e.g. when a long circular spring is wound around a turret as a support element. Adjacent primary structure elements may be contacting each other or even interfere with each other, such that effectively the total anvil drum surface may be covered by the primary helical structure, or they may be spaced apart from each other. Such a set up can be very advantageously used for example when a wide web is to be consolidated over its entire surface, e.g. to form pockets as described herein below. In a further particular execution, two or more helices (respectively parts of one helix, which may be wound around a cylinder) may be positioned adjacently to each other in a staggered or interdigitating arrangement.

A further particular execution of a helical anvil structure is a litz wire, illustrating the broad range of helical materials with regard to size of bonding points as resulting from small diameter strands, also with regard to a high area density of bonding points as may result from the high number of strands, and a high flexibility as being a typical characteristic of litz wires.

Apart from providing a suitable geometry, a helical element also exhibits very favourable flexibility properties. Such properties can also be equivalently provided by structures, which in the strict mathematical sense are not helices but can be considered as extensions of the term helix. In particular this relates to a two dimensionally formed wave-like wire, e.g. if a wire has a generally x-directional extension and is sinusoidally or otherwise ondulatingly deflected in the z-direction, but forms a straight line when looking at it in a z-directional top view. i.e. having no y-directional deflection. In particular when the wire cross-section has the appropriate rounded surface shape, such a wire element would satisfactorily perform as a helical element in the present context. In such an execution, the ondulated form functions as a flexibility providing element. Taking this equivalence even further, a straight wire may function satisfactorily, if it has the appropriately rounded cross-sectional shape and if it is supported by a flexible wire support member to exhibit the required flexibility.

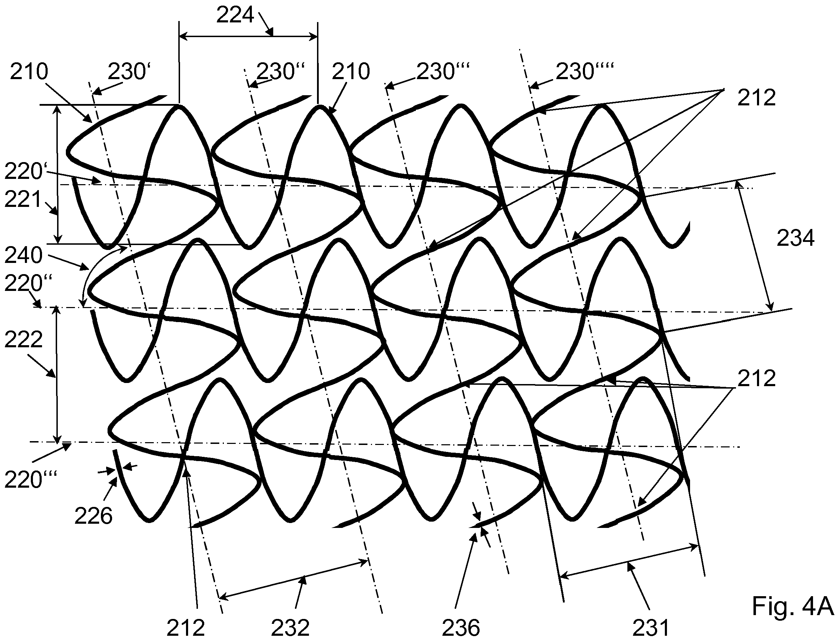

An exemplary execution of a set of interdigitizing helical springs can be seen in Fig. 4A schematically with explanatory lines and numerals. There is a first set of three springs 210 positioned parallel to each other along axes 220', 220" and 220'". They exhibit a wire diameter 226, a pitch length 224, an outer spring diameter 221, and are spaced apart by distance 222. A second set of four springs 210 is shown positioned parallel to each other along axes 230', 230", 230'", and 230"" which exhibit an angle 240 to the axes of the first set of springs, further a wire diameter 236, a pitch length 234, an outer spring diameter 231 and are spaced apart from each other by distance 232. The wires have upper portions 212 and lower portions 218, wherein "upper" and "lower" portions are based relative to a plane made of the axes of the system and relative to the position of a viewer. In the arrangement shown an upper portion of one set is positioned over a lower portion of the other set and vice versa.

Such helical elements provide the desired bonding patterns by serving as a first web support, e.g. as an anvil in the bonding system, which interacts with the energy source.

A first web support should satisfy certain mechanical requirements, such as flexibility.

A material is considered to be flexible, when it passes the flexibility test by exhibiting a vertical deflection in the test of more than 0.01 mm. Preferably, the material deflects more than 0.1 mm, more preferably more than 0.3 mm, and further suitable materials may exhibit a value of more than 1 mm or more than even 1 cm. It should be noted, that the flexibility test requires the deflection criterion to be satisfied in two directions perpendicular to each other. The skilled person will readily realize, that other test methods such as ISO 12135 (Metallic materials, Unified method for the determination of quasi-static fracture toughness), ASTM D790 (Standard test methods for flexural properties of unreinforced and reinforced plastics and electrical insulating materials), ISO 178: Plastics-Determination of flexural properties) aim for determining essentially the same property, and thus may be employed equivalently.

In a preferred execution of the invention the flexible anvil element exhibits a flexibility which is higher than the one of its support structure. Hitherto it has been believed that in order to provide good and uniform creation of consolidation regions, all parts of the equipment need to be sturdy and rigid, and any deformation should be minimized. Thus, heated calender rolls are often designed and manufactured towards minimal deformation, or ultra-sonic equipment has essentially undeformable anvils and complicated measures are taken to adjust the gap width upon material variability and / or process variability, such as temperature increases during operation.

This is further explained by considering a coil spring as a non-limiting example for such a helical element useful as a first web support means. The coil spring may be made from a type of steel having a modulus of elasticity which is lower than the one of its support member. However, the particular shape of the coil spring provides a much higher flexibility than the wire of the coil as such. Without wishing to be bound by the theory, it is believed that this is due to the following reasons. First, considering the arched structure of a wire turn, this may transmit the forces tangentially away, and some reversible deformation may deform for example a circular wire into a somewhat elliptic one when the compression occurs. Even further, neighbouring turns may move relative to each other. Thus, the system exhibits a particular robustness with regard to variability, as it can react differently for each and every consolidation region. This combined effect is believed to result in a significantly smoothed operation, and peak forces are buffered away. Preferably, the helical element is metallic, though thermo- and/or duroplastic materials are also contemplated, if these satisfy the mechanical and thermal requirements.

In addition to the function as a bonding anvil, the first web support means serves as a sandwich pocket forming element.

Thus the first web support means delimits accumulation regions from each other or - if present - from empty regions.

The term "delimits" refers to the situation that the contact surface pattern of the first web support - corresponding to the bonding pattern - forms pattern lines, as selected connecting lines of the contact surfaces, corresponding to the bonding indentations in the web.

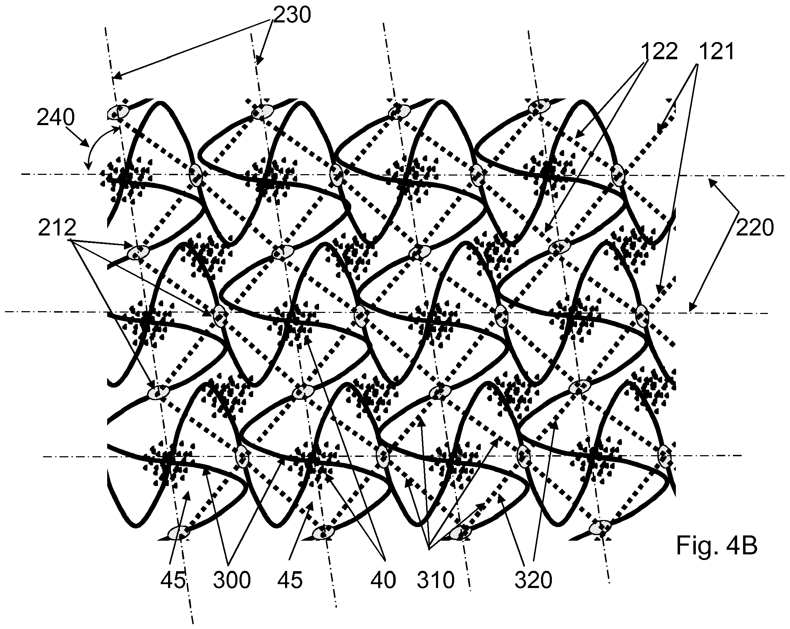

Thus the first web support means can be seen to form ridges or "dams" separating the "valleys" or "depths" of the accumulation regions. Considering for non-limiting explanatory purposes the schematic Fig. 4B, in which a helical element system as shown in Fig. 4A. In addition to the helical elements 210 along axes 220 and 230 with their respective "upper" portions indicated as ovals 212 oriented towards the web (which is omitted in the schematic illustration) and with accumulation regions 300 with free flowing material 40, positioned on the web material (not shown). The accumulation regions 300 represent z-directional deflections (or troughs or valleys) of the web between the ridges 310. The ridges 310 are formed by the upper portions 212 of the helical elements and their connections along pattern lines 121 and 122. Along these pattern lines, the web material is deflected somewhat in the z-direction as it is supported at the upper portions 212 but can be unsupported there between, thusly forming saddle points 320. Thus, the web forms z-directional deflections in the regions 45, where free flowing material is positioned in accumulation regions (300), circumscribed by ridges formed by the regions supported by the upper portions of the helical elements connected via the saddle points.

If free flowing material is deposited onto the web, it will accumulate in the accumulation regions based on gravity or it may be directed there by vacuum and/or air flows. Also if it would be applied by a simple doctor blade or brush system, it would flow or be scraped or brushed into the "valleys". Thus even a homogeneous application of free flowing material results in a patterned distribution thereof. Further, at least the upper portions, if not all the ridges, are essentially free of free flowing material. The skilled person will readily realize that also patterned or profiled pre-metering of the free flowing material can be performed, but it is not an essential aspect for executing the present invention.

As the regions as supported by the upper portions of the helical element are now essentially free of free flowing material, a cover, preferably a cover web, can be applied and bonded in these upper regions. Depending on the size of the pockets relative to the properties and amount of the free flowing material, the pockets can provide sufficient immobilization to the free flowing material.

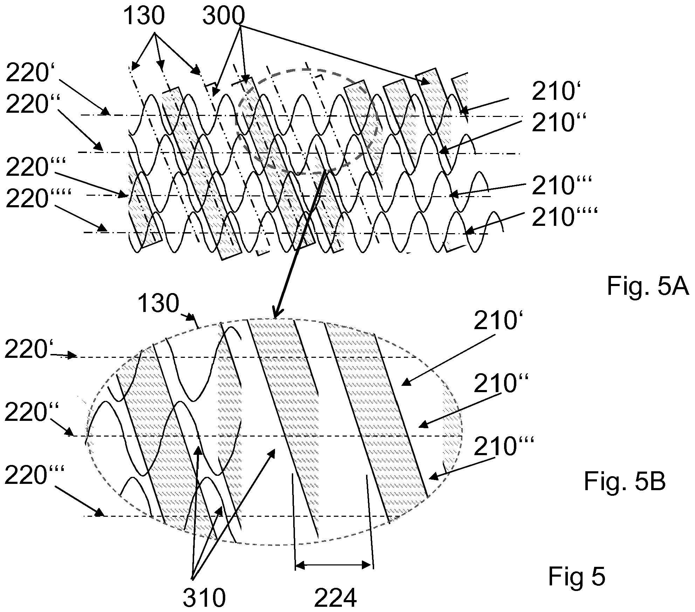

A further explanatory description for forming of accumulation regions in the z-directional deflections in a web material can further be followed by referring to Fig. 5A, with an enlarged view of a portion thereof in Fig. 5B. There are shown four helical elements 210, 210', 210"', and 210"", positioned equidistantly and parallel to each other such that they are almost in contact with each other and longitudinally offset by a pitch length. Each has a primary pattern line 220, which may be, but do not need to be, aligned with the length or machine direction, but which - in analogy to the definition used for bonding points - defines columns of the contact surfaces corresponding to the axes of the helical elements. If a carrier web is positioned over the helical elements, and z-directional deflection is induced such as by vacuum, the web will deform such that the regions of the contact surfaces will form ridges like dams, whilst the areas between these form the depth of elongated trough like accumulation regions 300, into which free flowing material can be deposited. Thus, in such an execution, each elongated valley is surrounded by pairs of ridges, formed by adjacent ridges of adjacent helical elements..

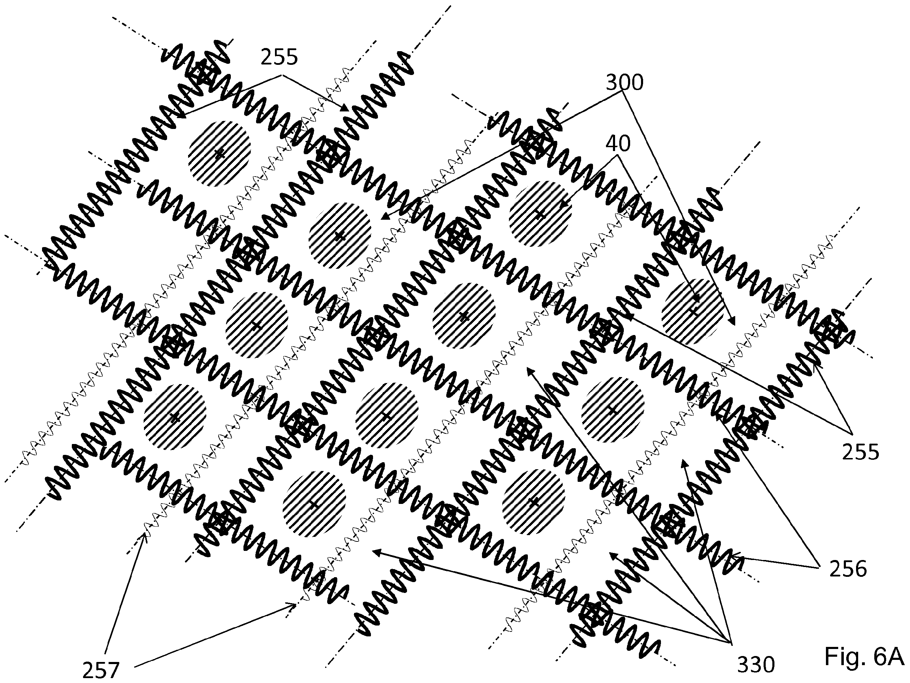

Fig. 6A depicts schematically another approach for creating ridges and valleys. Therein, a first set of helical elements 255 and a second set of helical elements 256 positioned at an angle to the first set form a cross-wise pattern of ridges with valley regions there between. When overlaying the carrier web, it will be supported by the first web support means. Optionally, the web may also be supported by a second web support means in the valleys, which may simultaneously be the deflection means, such as by allowing to create vacuum, such as through holes therein. Accordingly, free flowing material 40 can be positioned in the accumulation regions 300. The first web support means intercept each other at cross-points spaced apart more than one pitch length, such that each ridge is formed by two or more contact surfaces.

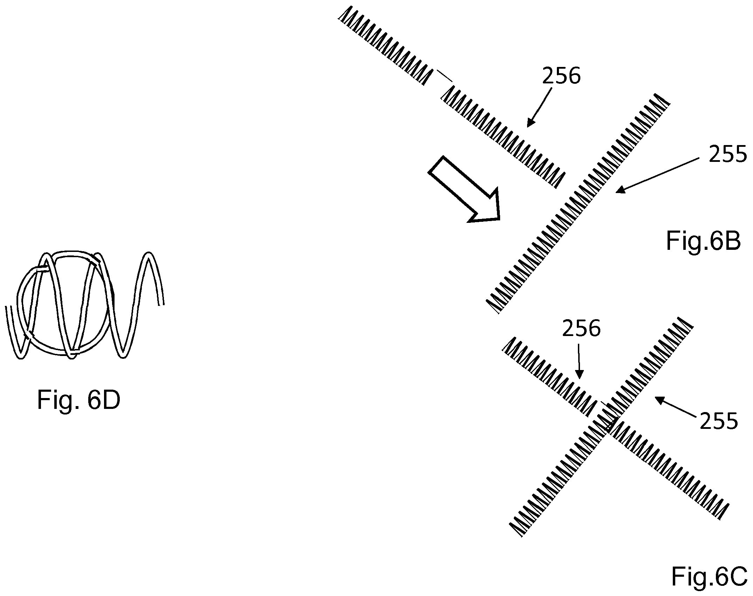

As it is desired to have the contact surfaces levelled, i.e. at essentially the same distance to the support means, the crossing of the helical elements may be done with interrupting the helical structure there, such as by stretching the helical element in this region before engaging the elements (see Fig. 6B and 6C). There may also other measures be taken such as forming indentations in the helical structure, as indicated in Fig. 6D.

Fig. 6A further exhibits exemplarily an execution with a third set of helical elements 257 of different size and/or shape, e.g. by having a different diameter, or pitch, or wire diameter, or flexural modulus. Optionally, such helical elements with a varying size may be supported by height compensation means to maintain the levelling of the contact surfaces.

Also shown in Fig. 6A is an execution of a sandwich structure with accumulation regions 300 as well as empty regions 330, which are surrounded by bonding regions 340, but which are essentially void of free flowing material. Combined with bonding lines exhibiting varying bonding strength, such as may be accomplished by varying size or shape of the helical elements, or flexural modulus, or preapplied perforations in one of the webs, this provides a particularly advantageous design for the free flowing material being superabsorbent particles, which may upon swelling may have more expansion space available.

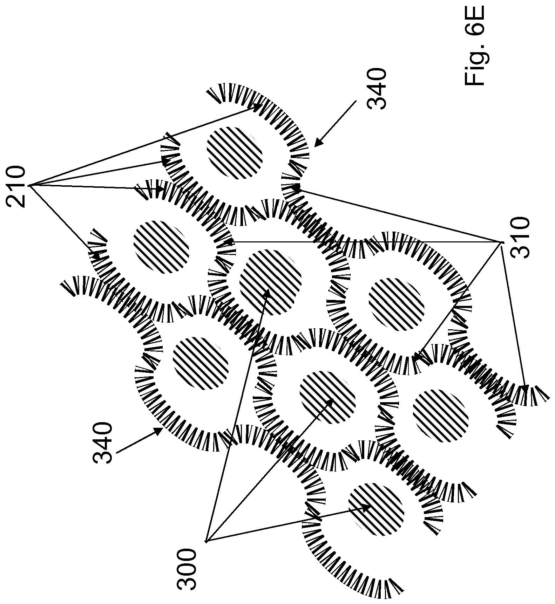

Fig. 6E shows schematically and non-limiting another execution for the arrangement of helical elements. Therein, the helical elements 210 are positioned along a curvilinear axis, such as a wavy or sinusoidal curve, which have a general parallel but offset arrangement, thereby forming pocket or accumulation regions 300 and ridges 310 circumscribed by bonding regions 330.

In yet a further particular execution, the helical elements are rotatably mounted, such that they can successively, such as element after element, or pair of adjacent elements after pair of adjacent elements, be rotated along their axis.

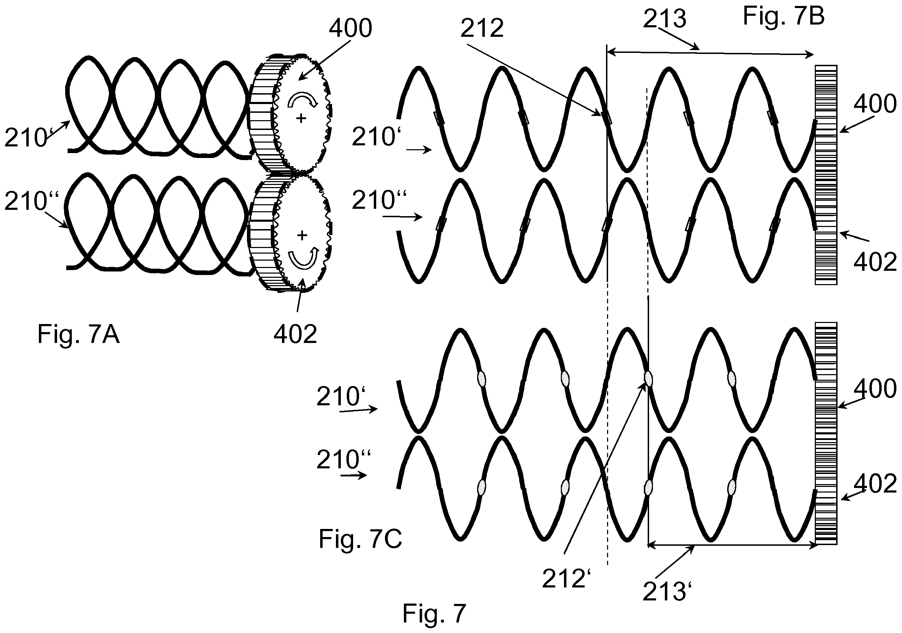

This can be achieved by positioning the helical elements at an angle to the MD such that they are not essentially endless elements but terminate at the periphery of the support element. As schematically indicated in Fig. 7A, helical elements 210' and 210" may be connected to rotary drive elements 400 and 402, which may be connected to the support element (not shown).

Upon activation of the rotation, the helical elements can either rotate along their axis with the axis and the rotary drive elements remaining stationary relative to the support, or the helical element can roll over the support, i.e. the axis as well as the rotary drive elements move translatorily.

In either case, the upper portions 212 of the helical element reposition relative to the support element.

The first case is depicted schematically in Fig 7A in a perspective and in Fig. 7B in a top view with a first set of upper portions, an exemplary one thereof 212 being positioned at a distance 213 from the rotary drive elements 400 and 402 respectively, prior to rotating. After a 90° rotation, as indicated in Fig. 7C the part of the helical element that had formed the upper portions has now moved downwardly, and the part of the helical element previously forming the upper portion 212 is now forming a lower region, and the next upper portion replacing 212 is now shown as point 212' at a different distance 213' to the rotary drive elements.

In the second case, the dislocation is not strictly along the helical element axis, but rather at an angle thereto. When a web is positioned over the helical elements prior to rotating, it contacts the helical element at the upper portions. Upon activating the rotating whilst holding the web stationary to the support, the "ridges" will move according to the move of the upper portions. Consequently, regions of the web that belonged to ridges before the rotation will become "valleys" thereafter.

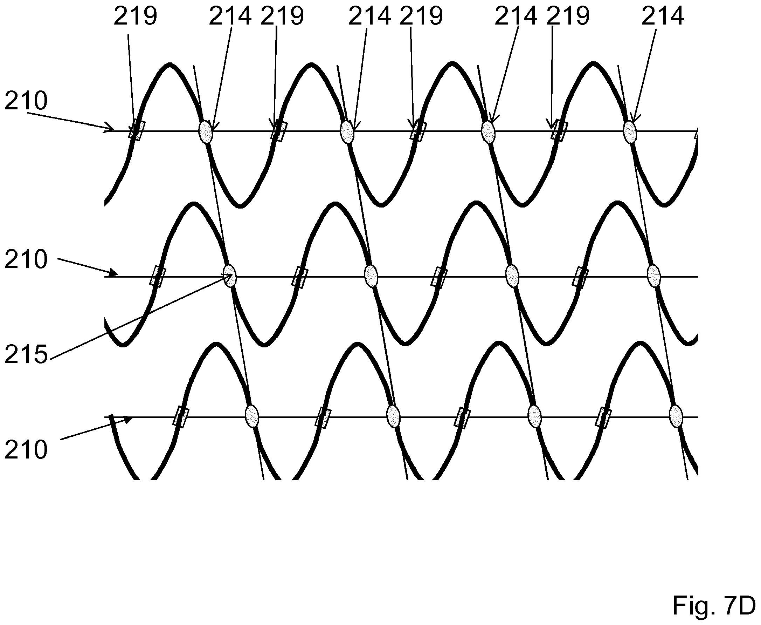

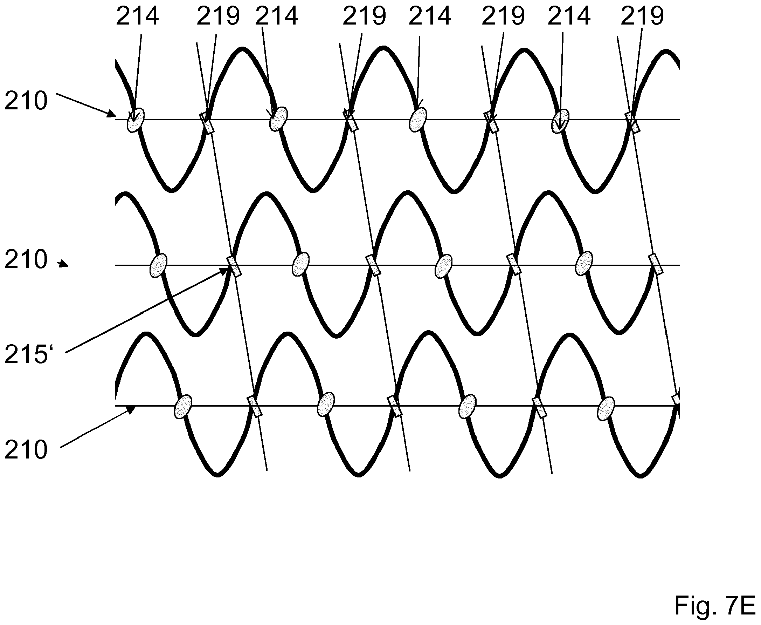

As shown schematically in Fig. 7D, three parallel positioned helical elements 210 have upwardly (i.e. towards the carrier web) positioned first surfaces 214 (marked in Fig. 7D, and 7E with ellipses) and downwardly positioned second surfaces 219 (marked with rectangles), as may rest on the support element. Upon rotating the helical elements by 180°, the first and the second surfaces exchange their positions relative to the carrier web respectively the support, see Fig. 7E. Accordingly, for a carrier web placed thereon, also the ridge and valley regions will change, e.g. a particular point 215 belongs to a ridge region prior to rotation (Fig. 7D) and as point 215' to a valley region thereafter (Fig. 7E). A particular non limiting application of this aspect can be to treat certain portions of the carrier web with an adhesive, whilst leaving other regions essentially free of adhesive. As it may be preferred to have some adhesive properties in e.g. the valley regions, for example to at least partially increase the immobilization of the free flowing material especially in the case it is particulate, but it may also be preferred to have no adhesive in the contact surface upon bonding, e.g. for ultra-sonic bonding.

To this end, adhesive is applied to the web essentially only to the first surface regions, i.e. the ridges prior to a rotation, such as by vacuum or air flow guided glue spray or by application via a so called "kiss roll" onto the first regions but not onto the valley regions centred over the second regions. Then, the helical elements are rotated. Thereupon, the "ridge" regions will dislocate without dislocation of the web, and the web regions positioned there over prior to rotation will transform into valley regions thereafter. Correspondingly, the essentially adhesive free portions of the web from the valley during adhesive application will then be supported by the upwardly positioned portions of the helical element, now forming the contact surface for supporting the (adhesive free) web portion during the ultrasonic bonding.

A sandwich structure according to the present invention is formed on a sandwich forming element. The sandwich forming element comprises an essentially endless surface, which is moveable in its machine direction and exhibits a CD-width. The sandwich forming element comprises a support structure on which the first web support element can be mounted.

In a first embodiment, this support structure may be an essentially endless belt on which the first web support means rests. In a second, often preferred execution, as schematically presented in Fig. 3A, the support structure 90 is a rotating drum, with both executions being well known in the art. The support structure may have a flat, in case of a turret a cylindrical surface, and the first web support means may simply be positioned on this surface.

Optionally, the support structure may have indentations or recessions for receiving the first web support means. Optionally, there may be an interim layer, such as a height adjustment or a damping or flexible layer between the support structure and the first web support means. Optionally there may be first web support shaping means connected to the support structure or the sandwich forming means, such as described in above referenced WO'055 publication.

Optionally, and often preferably, the sandwich forming means may have further means for enhancing the deflection of the carrier web, such as may be achieved by applying vacuum.

In one execution, all of the surface of the support structure may be air permeable, such as by having holes or perforations. Optionally, the air permeability may vary over the surface, and may be adapted to the shape of the first web support means.

In a particular execution, the air permeability may be restricted to the regions of the first webs support means, or parts thereof.

Another way to enhance the deflection of the carrier means is to "overfeed" the web to the support structure, i.e. to feed it to the surface of the structure at a web speed which is higher than the surface speed of the support structure. The web will then pucker up, i.e. tend to form wrinkles or folds, which - optionally aided by a pucker guide and/or the deflection means - extend into the valleys of the web support means. In the cases as depicted in Fig. 4 and Fig. 5, the depth of the valleys is limited by the inner diameter of the helical elements. In case as depicted in Fig. 6 the web may form pockets which may additionally be supported by a second web support means in the valleys.

Thus having described structural features of the elements of the present invention, it is now shown, how the free flowing materials can be sandwiched between extensible webs. The carrier web is supplied to the sandwich forming means such that its MD is aligned with the MD of the forming means.

The carrier web is positioned on the contact surfaces of the first web support means. Due to small z-directional forces, the carrier web will be deflected between individual contact surfaces. Some z-directional forces may - in case of the sandwich forming means being in the form of a turret - already be induced by applying a certain web tension. For a flat belt system, or alternatively or additionally for a turret, vacuum suction may be created through the surface of the sandwich forming means.

Depending on the intended use, the deflection may be so deep, that the carrier web contacts a second web support surface, as may be formed by the surface of the support structure of the sandwich forming means. This may further be enhanced by deflection aids, such as vacuum suction, or so called puckering means or other means creating ondulations in the web.

Upon appropriate shaping of the first web support means, optionally in combination with adaptation of the deflection aids such as vacuum suction, there may be regions or valleys with more or with less deflection, or even regions without any deflection.

Upon forming of the valleys, the free flowing material can now be positioned on the at least partly deflected carrier web. Whilst the particular method of doing so is not of high importance for executing the present invention, the present invention allows the use of simple methods. In particular for many applications no pre-metering, which is important for most other sandwich forming methods, in particular for high speed applications, is required. This applies both for even distribution of the free flowing material over the pattern, but also for varying patterns with a different degree of accumulation thereof, as can be readily achieved by defining pockets / valleys of various shapes and sizes over the sandwich structure

In one execution, applicable both for fluid or particulate materials, a curtain of the free flowing material may be constantly fed to the sandwich forming means, and then, optionally guided by air streams, distributed into the valleys. In a particular execution, the particle stream 510 may be modulated so as to provide a varying distribution of particles upon lay down. This modulation may be by any means resulting in a variation across the y-direction and/or the x-direction. In particular the latter may be achieved by interrupting the particle stream, such as by blowing or sucking air and thus removing the particles from the stream at a predetermined rate for a predetermined time. A very convenient and simple construction employs a vacuum cylinder rotating around its axis which is positioned between the SAP metering device and the rotating drum 720 in the proximity of the particle stream and oriented along the y-direction. The cylinder further comprises a slit of a predetermined width, such that when the slit is oriented towards the particle stream, the particles are sucked into the cylinder, from where they may be recycled to the SAP particle supply system, but alternatively they may be fed towards the rotating drum on which already a carrier material is positioned, such that in such a predetermined region an increase of SAP basis weight can be realized.

In another execution, the free flowing material is furnished in a trough, which - especially in the case of particulate free flowing material - may be a fluidized bed. The sandwich forming element is positioned above the trough (relative to gravity) and arranged such that it immerses into the free flowing material. Upon application of vacuum, the free flowing material will then be filled into the deflection regions.

Another execution may employ the well known doctor blade or brush system, where all valleys are first overfilled and then scraped or brushed off to a filling level. Various such systems for particulate material are described in EP1621165 (P&G, Blessing), to which express reference is made herewith. In EP1621166 (P&G, Blessing) a system is described employing a premetering system. Similarly, WO2012/048878A (Romanova; van de Maele) describes an accumulation regioning means.

In a particular execution, all deflections will be filled to the same height, and depending of the size and depth of the deflection or valley, the accumulation regions may contain essentially the same or varying (by weight or by volume) amounts of material, or even empty regions may be formed.

The distribution of the material may further be guided by vacuum or air streams, especially for particulate free flowing material. For example, such vacuum is not active in the contact areas of the web to the element supporting it, such that the free flowing material will not accumulate in those contact areas.

For certain applications, it may be preferable to have the accumulation regions completely filled, for others an incomplete filling may be preferable.

Based on the particular arrangement of the first web support means creating ridges and valleys, the ridges can much easier be kept free of the free flowing material.

Once the free flowing material is positioned on the carrier web, the cover web can be supplied to and positioned onto the carrier web, such that it contacts the carrier web at least in the regions corresponding to the contact surfaces of the first web support means. Optionally, two different free flowing materials, such as particles and fibres, may be added simultaneously or consecutively.

Optionally, adhesive may be applied to either of the webs prior to the combination of the webs. The composite of carrier web and cover web with the free flowing material positioned there between is then fed towards the bonding means, which forms a gap with the first web support means for compressing the webs together.

In the case of pre-applied adhesive, adhesive bonding points will be formed according to the shape of the contact surfaces of the first web support system forming the final bonding pattern.

In the case of energy application, the carrier and the cover web can be melt-fusion bonded, in a preferred execution by ultra-sonic energy.

The final bonding pattern will be created, both with regard to its shape but also with regard to its bonding strength depending on the
- design of the first web support system, in particular of the contact surfaces,
- the shape of the energy source,
- the number of the energy sources, and the relative positioning in case of more than one,
- the operation of the energy source (e.g. constant or intermittent).

This final bonding pattern will then circumscribe the pockets with or without free flowing material.

The method described herein, not part of the present invention, is particularly suitable for producing sandwich structures at high production volumes such as at production speeds with web speeds of more than 100 m / min, preferably more than 300 m /min or even more than 700 m / min.

Whilst the description so far should be seen to only explanatorily and not limiting describe the features of the present invention, the following describes particular applications for the present invention.

An area, for which the present invention provides particular advantages, is the use for absorbent articles, such as - without limitation - baby diapers. Such articles comprise superabsorbent material in particulate form. Recently, so called "airfelt free" structures have gained interest, such as described in the above mentioned EP'166, EP'165, or WO'878. The present invention provides an improvement over the articles resulting from the process described therein. In a first example, a first web support means is formed according to Fig. 4. The helical elements are helical steel springs exhibiting an outer diameter of from 5 mm to about 35 mm, with a diameter of 15 mm working well. The wire diameter may be from about 0.2 mm to about 5 mm with about 1.5 mm working well.

The pitch length of the helical spring can be from about 3 times the wire diameter to about 30 or even 50 times the wire diameter. For the above mentioned wire diameter of 1.5 mm, a pitch length of about 17 mm works well.

These springs are appropriately interdigitizingly meshed / crossed and cover essentially all of the surface of the sandwich forming means.

The sandwich forming means can be a vacuum turret having a circumference of e.g. 700mm or 1400 mm, corresponding to the length of two or four absorbent cores of an absorbent article each of a length of 350 mm, a width of 100 mm corresponding to the width of an absorbent core of an absorbent article of e.g. 120 mm, and an air permeable surface, such as a mesh with openings of 2 mm. Alternatively, a perforated metal sheet or an air permeable sinter material can be used.

The openings or the perforations may be plugged or closed by appropriate means so as to create regions of increased, reduced or no air suction. Optionally, though implying an increased complexity, certain areas may actually be connected to an air blower so as to create overpressure instead of a vacuum.

For the application in disposable absorbent articles, the webs should exhibit a flexibility and pliability to not negatively impact the feel to the touch or haptic properties. At least one of the carrier and cover webs should be liquid permeable. Preferred non-woven materials are provided from synthetic fibres, such as PE, PET and most preferably PP. As the polymers used for nonwoven production are inherently hydrophobic, they are preferably treated with hydrophilization coatings. With conventional and commercially available superabsorbent material, exhibiting a typical particle size distribution, both the carrier and the cover web can be a conventional nonwoven material, such as a 17 g/m² spunbond-meltblown-spunbond polypropylene materials.

Either one or both of the carrier web and the cover web may further provide liquid acquisition and/or distribution functionality in the absorbent article. Typically this is achieved by modifying the pore size respectively pore size distribution in the material. For liquid acquisition performance, larger pores are desired so as to not impede liquid flow there through. For liquid distribution smaller pore sizes are desired so as to create an adequate wicking effect. Such pore sizes can suitably be designed by introducing different types of fibres. Often it is preferred to have materials with a layered structure, such that for example a top layer may have large pores for liquid acquisition performance overlying another layer with smaller pores. Thus the webs can comprise a variety of fibrous materials, such as naturally occurring fibres (modified or unmodified), as well as synthetically made fibres. Examples of suitable unmodified/modified naturally occurring fibres include without intending any limitation cotton, wood pulp, or chemically modified wood pulp, and rayon. Whilst it is important that the cover and the carrier layer comprise at least together sufficient amount of components exhibiting compatible melt properties, other synthetic fibres may be included, such as made from polyvinyl alcohol polytetrafluoroethylene, polyacrylics such as ORLONIO, polyamides such as nylon, and the like. Whilst polyolefins such as polyethylene (e.g., PULPEX) and polypropylene may be preferred when considering melt properties, in particular polyesters materials such as DACRON* or KODELO have been found to provide good performance as acquisition / distribution material. Also suitable in this context are bicomponent fibres, such as sheath/core fibres combining polyethylene/ polypropylene, polyethylene/polyester, polypropylene/polyester, and the like. Suitable bicomponent fibres for use herein can be either uncrimped (i.e. unbent) or crimped (i.e. bent). Typically such fibres can have a thickness in the range from less than about 1.0 dTex to about 20 dTex.

The carrier or cover webs with combined liquid acquisition / distribution functionality should also prevent particles of free flowing material from penetrating through, even though they should exhibit a sufficiently open pore structure by having at least a layer with pore sizes of more than about 200 µm, preferably more than 300 µm, as can be determined by using a Coulter 115/60 porometer from Coulter Electronics, Ltd. of Luton, England, or equivalent. The retention of free flowing material can be achieved by having a layer with sufficiently small pores to retain the particles in analogy to a surface filter. For certain applications it may be advantageous to have the free flowing material particles to penetrate into the pores of the web material and retaining them in analogy to a depth filter. This may become particularly effective in absorbent articles, if the carrier web is such an open pore web for receiving superabsorbent material particles in its pores. Further suitable web materials for being used as carrier or cover material are films, under the proviso that at least one of the webs is sufficiently liquid permeable. Such films may be a plastic film such as a thermoplastic film having a thickness of about 0.012 mm to about 0.05 mm, as often used as so called backsheet films for the use in disposable absorbent articles. Optionally such films may permit vapours to penetrate. Other suitable film materials may comprise apertures such as described in US5571096 (P&G; Dobrin, formed films).

The carrier or cover material may comprise liquid handling enhancing means, such as materials that enhance liquid distribution and/or interim liquid storage capability (i.e. they receive liquids within their structure and release all or a portion thereof to ultimate storage means, such as superabsorbent polymers, optionally at different locations than where they received the liquid). Such liquid handling enhancing means may be conventional cellulosic tissues, may comprise chemically stiffened, twisted and curled cellulosic fibres, or may comprise particular combinations of synthetic fibres, such as hydrophilized micro fibres like meltblown fibres. Particular executions are described e.g. in WO1999/055266, to which express reference is made with regard to liquid distribution materials as liquid handling enhancing means.

Such a liquid handling enhancing means may comprised in the carrier and/or the cover material as these are delivered to the process, or they may be combined in-situ, i.e. they may be fed to the process separately and combined just prior to the combination with the free flowing material. If the liquid handling enhancement means of the carrier and/or cover material is present in the form of a discernible layer, this may be positioned adjacently to the free flowing material or may be positioned towards the outer surface of the resulting sandwich structure.

In order to determine if the carrier and cover materials have sufficient retention capability for free flowing material particles, the following test may be performed. As free flowing material a conventional superabsorbent material of the broken gel type is selected and sieved according to EDANA test method WSP 220.3 (11). 0.5 g of the sieve fraction between 90 µm and 149 µm are placed on a sample of 200 mm by 100 mm of the cover or carrier material. The sample is folded over onto itself to cover the particles, forming a 100 mm by 100 mm packet. The sides of the packet are closed by a piece of adhesive tape, which overlaps the packet periphery by approximately 0.5 mm. The sample is now attached in a Model RX-24 Shaker from the Tyler Company or equivalent, operating at about 520 cycles per minute with a stroke of about 1.25 cm. Below the sample there is placed a tared pan of sufficient size to collect any superabsorbent shake-out. The shaker is turned on and operated for a period of five minutes. Then, the pan is reweighed and the difference between the collected weight and the tared weight represents the amount of shake-out. Preferably, the materials show a shake out of less than 10 weight-%, more preferably of less than 5 weight-%, and most preferably of less than 1 weight-%.

When such a material is positioned over the turret and usual web tension is applied, the pockets will start to shape. This shaping gets more pronounced upon the application of the vacuum.

The sandwich structure may conveniently comprise superabsorbent material, often also referred to as "absorbent gelling material" or "AGM", "absorbent polymer material", which are typically, though not necessarily partially cross-linked polymeric materials, which can absorb water whilst they are swelling to form a gel. Typically, these are in irregularly shaped or spherical granules, which can swell upon contact with liquids, such as urine. Whilst this material may be in various shapes or forms, such as granular, spherical, flakes, fibrous, it will often consist of irregularly shaped particles, having a mean particle size of from 10µm to 1000 µm, preferably with less than 5% by weight having a particle size of 5 µm, and preferably with less than 5% by weight having a particle size of more than 1200 µm.

Preferably superabsorbent material useful in disposable absorbent articles should not substantially obstruct the liquid flow throughout the material, even in the swollen state, i.e. when liquid has been absorbed. Thus, preferably the material exhibits a liquid permeability as expressed by the Saline Flow Conductivity of the absorbent polymer material of more than 10, 20, 30 or 40 SFC-units, where 1 SFC unit is 1 x 10⁻⁷ (cm³ x s) / g. Saline flow conductivity is a parameter well recognized in the art and can be measured in accordance with the test disclosed in EP 752 892 A1 (Goldman et al; P&G).

The superabsorbent particles are sprinkled onto the surface of the carrier web by a particle curtain at a rate delivering the desired absorption capacity, which evenly distribute into the created valleys, thusly forming the pockets of the sandwich structure.

A sandwich structure which is particularly useful for disposable absorbent articles such as diapers should provide good immobilization or the absorbent material, both in a dry and in a wet state. Preferably the sandwich structures or the absorbent article comprising such structures should provide more than 50%, preferably more than 60%, even more preferably more than 80% and most preferably more than 90% when tested in the Wet Immobilization Test, such as described in EP1447066 (Busam et al.; P&G). In addition, the structure should not be too stiff or rough, and should provide a high liquid retention capacity.

The accumulation zones with superabsorbent material may comprise as few as about 10 particles, but may also be up to several hundred or even several thousands of particles. The particles may be arranged essentially in a "monolayer" arrangement, or multi-layer structures of essentially constant thickness, or may have varying thickness. Typical basis weights as averaged over an accumulation region range from 10 g/m² or even less to 500 g/m² or even 1000 g/ m². Typical basis weights averaged over a primary pattern (i.e. including both accumulation regions and the regions between these, i.e. empty regions and bonding regions) range from about 1 g/m² to more than 400 g/m² or even more than 800 g/m².

The accumulation regions may have various forms and shapes, such as oval or elliptic. In a particular execution, these accumulation regions may be approximately circular, exhibiting a diameter of more than 2 mm, preferably more than 4 mm, but less than 30 mm, preferably less than 8 mm. The distance between two neighbouring accumulation regions in a pattern may be more than 2 mm, preferably more than 5 mm, but less than 30 mm, preferably less than 15 mm, and even more preferably less than 7 mm.

A plurality of accumulation regions is forming a primary pattern wherein the accumulation regions are spaced apart in any geometric way. Such a pattern may comprise as few as two accumulation regions, but will typically comprise more than ten accumulation regions. Often, it will comprise less than 1000 accumulation regions. Any plurality of accumulation region may form regular or irregular sub-patterns of the primary pattern. The accumulation regions may be discrete or disconnected regions, such that each of these regions is essentially circumscribed by a region which is essentially free of particles.

### Bonding

The corresponding cover web is applied, and the composite is fed to a gap formed with a conventional sonotrode, such as Sonotrode Type MS 58/30/13 (Titanium; frequency 35 kHz, Lambda ½ Type with respective Ultrasonic Generator DYNAMIC digital control 600 CS) of Herrmann Ultraschall, Germany. If the width of one sonotrode is not sufficient, two or more thereof can be positioned next to each other, possibly in a slightly staggered arrangement to minimize edge effects. Optionally a rotating sonotrode, such as described in US20050034820 (Herrmann Ultraschall, Schneider) may be employed.

The bonding regions between the accumulation regions comprise preferably distinct bonding points rather than continuous regions. The bonding points may have varying shape and dimensions. In a preferred embodiment, the bonding points have an elliptic shape with a smaller width of not less than about 0.2 mm, preferably not less than 2 mm, or even not less than about 5 mm and a larger length dimension of not less than 0,25 mm, preferably not less than about 5 mm or even not less than about 10 mm. Further, the bonding point length or width is preferably less than about 30 mm, preferably less than about 20 mm. The ellipses have preferably an aspect ratio of length to width of between 1.05 : 1 and 200: 1. The distance of adjacent bonding points is primarily defined by the geometry of the helical element system, and preferably is not more than about 20 mm, preferably less than about 10 mm, more preferably less than about 5 mm.

The result is a sandwich structure with evenly sized and filled pockets with superabsorbent materials.

If desired, two sandwich formers can be positioned in a facing arrangement, so as to form a double sandwich with matching, or non-matching patterns, either of which can have their own cover sheet, and optionally may be bonded, or which share one and the same cover sheet for a single bonding.

## Claims

1. A sandwich structure with free flowing material between webs,
said sandwich structure exhibiting a length (x-), width (y-) and thickness (z-) direction and comprising a carrier web (20) and a cover web (30), and particulate free flowing material (40) positioned z-directionally between said webs in x-y-directionally extending accumulation zones; wherein said carrier web and said cover web are bonded by melt fusion bonding by a bonding pattern,
and wherein said bonding pattern is formed by individual bond points (100) circumscribing said accumulation zones and wherein said bond points have an elliptical shape having an aspect ratio of long to short dimension of at least 1.05:1.

2. A sandwich structure according to claim 1, wherein said bonding of said webs is ultra-sonic bonding.

3. A sandwich structure according claim 1 or 2, wherein said bonding patterns exhibit one or more of the following:
- bonding points exhibiting an axis angle to the MD of less than 90°;
- curvilinear bonding pattern lines;
- two or more sub-pattern, wherein optionally the bond points of at least two of said sub-pattern exhibit different bond strength;
- said bonding pattern lines form pocket regions with free flowing material comprised in pockets corresponding to such regions, optionally with varying amount of free flowing material across various pockets;
- said bonding pattern lines form pocket regions which are essentially free of free flowing material.

4. A sandwich structure according any of claims 1 to 3, wherein said carrier or cover material comprises fibrous material comprising a layer having a median pore size of at least 200 µm.

5. A sandwich structure according to claim 4, wherein said carrier or cover material exhibits a superabsorbent material particle shake out of no more than 20 weight-%.

## Patentansprüche

1. Sandwichstruktur mit frei fließendem Material zwischen Bahnmaterialien, wobei die Sandwichstruktur eine Länge (x-), eine Breite (y-) und eine Dicke (z-) aufweist und eine Trägerbahn (20) und eine Abdeckbahn (30) umfasst, sowie frei fließendes Material (40), das z-richtungsmäßig zwischen den Bahnmaterialien in xy-richtungsmäßig verlaufenden Akkumulationszonen positioniert ist,
wobei die Trägerbahn und die Abdeckbahn durch Schmelzverbindung in einem Bindungsmuster verbunden sind,
wobei das Bindungsmuster durch individuelle Bindungspunkte (100) gebildet wird, die die Akkumulationszonen umgrenzen, und wobei die Bindungspunkte eine elliptische Form haben mit einem Seitenverhältnis von langer zu kurzer Dimension von mindestens 1,05: 1.

2. Sandwichstruktur nach Anspruch 1, wobei das Schmelzverbindung der Bahnen Ultraschallverbindung ist.

3. Sandwichstruktur nach Anspruch 1 oder 2, wobei die Bindungsmuster eines oder mehrere der folgenden Merkmale aufweisen:
- Bindungspunkte, die einen Achsenwinkel zu der MD von weniger als 90 ° aufweisen;
- krummlinige Verbindungslinien;
- zwei oder mehr Teilmuster, wobei gegebenenfalls die Bindungspunkte von mindestens zwei des Teilmusters eine unterschiedliche Bindungsstärke aufweisen;
- die Verbindungsmusterlinien Taschenbereiche mit frei fließendem Material in diesen Taschen bilden, die solchen Bereichen entsprechen, gegebenenfalls mit variierender Menge an freifließendem Material in verschiedene Taschenbereichen;
- die Verbindungsmusterlinien Taschenbereiche bilden, die im Wesentlichen frei von frei fließendem Material sind.

4. Sandwichstruktur nach einem der Ansprüche 1 bis 3, wobei die Trägerbahn oder die Abdeckbahn faseriges Material umfasst, das eine Schicht mit einer mittleren Porengröße von mindestens 200 µm umfasst.

5. Sandwichstruktur nach Anspruch 4, wobei die Träger- oder Abdeckbahn einen SAM-Partikel-Shake-out von nicht mehr als 20 Gew .-% aufweist.

## Revendications

1. Structure sandwich avec un matériau s'écoulant librement entre des bandes, ladite structure sandwich présentant une longueur (x-), une largeur (y-) et une épaisseur (z-) et comprenant une bande porteuse (20) et une bande de couverture (30), et une matière s'écoulant librement (40) z- directionnellement entre lesdites bandes dans des zones d'accumulation s'étendant dans la direction xy,
ladite bande de porteuse et ladite bande de couverture étant liées par un motif de liaison,
dans laquelle ledit motif de liaison est formé par des points de liaison individuels (100) circonscrivant lesdites zones d'accumulation
et en ce que lesdits points de liaison elliptique ont un rapport allongé au moins 1,05: 1.

2. Structure sandwich selon la revendication 1, dans laquelle ladite liaison desdites bandes est une liaison ultra-sonique.

3. Structure en sandwich selon la revendication 1 ou 2, dans laquelle lesdits motifs de liaison présentent un ou plusieurs des éléments suivants:
- des points de liaison présentant un angle d'axe au MD inférieur à 90 °;
- des lignes de motifs de liaison curvilignes;
- deux sous-motifs ou plus, dans lesquels, facultativement, les points de liaison d'au moins deux desdits sous-motifs présentent une force de liaison différente;
- lesdites lignes de motif de liaison forment des régions de poche avec ledit matériau s'écoulant librement et comprises dans des poches correspondant à de telles régions, facultativement avec une quantité variable de matériau s'écoulant librement dans diverses poches;
- lesdites lignes de motif de liaison forment des régions de poche qui sont essentiellement exemptes de matière s'écoulant librement.

4. Structure en sandwich selon l'une quelconque des revendications 1 à 3, dans laquelle ledit matériau de porteuse ou de couverture comprend un matériau fibreux comprenant une couche ayant une taille de pore médiane d'au moins 200 µιη.

5. Structure sandwich selon la revendication 4, dans laquelle ledit matériau de porteuse ou de recouvrement présente une secousse de particules SAM ne dépassant pas 20% en poids.
